# EUROPEAN PATENT APPLICATION

(11) **EP 2 244 027 A1**
(43) Date of publication of application: **27.10.2010**
(21) Application number: 09702778.3
(22) Date of filing: 05.01.2009
(51) Int. Cl.: F24F 6/12, A61L 9/14, B05B 17/06, C02F 1/46, F24F 6/00

(54) **SPRAY APPARATUS**

(30) Priority: 16.01.2008 JP 2008006924
(71) Applicant: SANYO Electric Co., Ltd., Moriguchi-shi Osaka 560-8677 (JP); Sanyo Consumer Electronics Co., Ltd., Tottori-shi Tottori 680-8634 (JP)
(72) Inventor: KANAMORI, Yoshiaki, Hyogo 657-0855 (JP); KANO, Kenzo, Gunma 370-0514 (JP)
(74) Representative: Glawe, Delfs, Moll
(86) International application number: PCT/JP2009/050492
(87) International publication number: WO 2009/091009

(57) **Abstract**

An atomizing apparatus 10 includes a housing (12), and a tank 40 stores tap water inside the housing. By electrolyzing the tap water by electrolysis electrodes (120p, 120m), the water in the tank becomes electrolyzed water. The electrolyzed water is guide upward through a feedwater rod 56, and supplied to an ultrasonic wave vibrator 76. By rotating or turning a lid 16 to an on-position, an emitting opening 24 and an opening 80 are communicated with each other, to allow mist to be emitted to the outside through the openings.

## Description

### TECHNICAL FIELD

The present invention relates to an atomizing apparatus. More specifically, the present invention relates to an apparatus emitting or diffusing mist of water by utilizing an ultrasonic wave vibrator.

### BACKGROUND ART

An example of such a kind of conventional apparatus is disclosed in a Japanese Patent No. 2858853 [F24F 6/00] (Patent Document 1).

The related art has a configuration of pouring water in a tank, and emit generated mist by a blow as necessary in order to guide it to the outside. Thus, this takes no account of carryability, movability and portability when water is in the tank.

### SUMMARY OF THE INVENTION

Therefore, it is a primary object of the present invention to provide a novel atomizing apparatus.

Another object of the present invention is to provide a portable atomizing apparatus.

The present invention employs following features in order to solve the above-described problems. It should be noted that reference numerals and the supplements inside the parentheses show one example of a corresponding relationship with the embodiments described later for easy understanding of the present invention, and do not limit the present invention.

A first invention is an atomizing apparatus comprising: a housing; a liquid storage which stores liquid for humidification within the housing; a liquid supplier which supplies the liquid stored in the liquid storage upward; a mist generator which is provided at an upper portion of the housing, and is supplied with the liquid from the liquid supplier to generate mist from the liquid; an opening which is formed above the mist generator, and passes the mist generated by the mist generator; a lid which is provided to be rotated between an on-position and an off-position at an upper end of the housing, and an emitting opening which is provided on the lid, and communicated with the opening when the lid is rotated to the on-position, wherein the mist is emitted through the opening and the emitting opening.

In the first invention, an atomizing apparatus (10: the reference numeral designating a corresponding portion in the embodiments. The same is true for the following description.) includes a housing (12), and a liquid storage such as a tank (40) stores liquid for humidification, for example, water in the housing. A liquid supplier such as a feedwater rod (56) supplies the liquid stored in the liquid storage to a mist generator (76) provided at an upper portion of the housing. The mist generator is supplied with the liquid and generates mist from the liquid. The mist generated by the mist generator is emitted through an opening (80, 94) and an emitting opening (24) formed on a lid (16).

A second invention is according to claim 1, and is an atomizing apparatus further comprising a fixing plate which is provided below the lid at the upper part of the housing; a first recessed portion and a second recessed portion which are provided on one of a top surface of the fixing plate and an undersurface of the lid at positions spaced apart from each other, and a protruded portion which is provided on the other of the top surface of the fixing plate and the undersurface of the lid, and is engaged with the first recessed portion when the lid is in the on-position, and is engaged with the second recessed portion when the lid is in the off-position.

In the second invention, a fixing plate (90) is provided below the lid at the upper part of the housing. A first recessed portion (98a) and a second recessed portion (98b) are provided on one of a top surface of the fixing plate and an undersurface of the lid (16), that is, the lower surface plate (100) at positions spaced apart from each other. A protruded portion (104b) is arranged on the other of the top surface of the fixing plate and the undersurface of the lid (16), that is, the lower surface plate (100). When the lid is placed in the on-position, the protruded portion (104b) is engaged with the first recessed portion, and when the lid is placed in the off-position, the protruded portion (104b) is engaged with the second recessed portion. According to the second invention, the lid is rotated between the on-position and the off-position with an operational feeling of click.

A third invention is according to the second invention, and is an atomizing apparatus further comprising a communicating member which provides communication between the first recessed portion and the second recessed portion, wherein the communicating member includes a slanting side being inclined inwardly near the first recessed portion and the second recessed portion and a connecting side connecting the slanting sides, further comprising a follower which follows the communicating member by displacing the protruded portion.

In the third invention, a communicating member (96) such as a rotating guide provides communication between the first recessed portion and the second recessed portion. The communicating member (96) includes slanting sides (96a, 96b) and a connecting side (96c), and therefore, the follower (108), such as a protruded portion being displaceable by a spring, moves along the communicating member when the lid (16) is rotated. In the third invention, the communicating member guides the protruded portion, resulting in a secure rotation of the lid.

A fourth invention is according to the first invention, and is an atomizing apparatus further comprising a switcher being operated when the lid is placed in the on-position.

In the fourth invention, when the lid (16) is placed in the on-position, the switcher (110, 112) is activated to make the atomizing apparatus (10) place in an operating state. According to the fourth invention, by placing the lid (16) in the on-position, it is possible to perform an operation.

A fifth invention is according to the fourth invention, and is an atomizing apparatus further comprising a fixing plate which is provided below the lid at an upper part of the housing, wherein the switcher includes a switch which is provided on one of a top surface of the fixing plate and an undersurface of the lid and has an actuator, and an acting portion which is provided on the other of the top surface of the fixing plate and the undersurface of the lid and acts on the actuator when the lid is placed in the on-position.

In the fifth invention, a fixing plate (90) is provided below the lid at an upper part of the housing. A switch (110) is provided on one of a top surface of the fixing plate and an undersurface of the lid (16), that is, the lower surface plate (100), and an acting portion (112) is provided on the other of the top surface of the fixing plate and the undersurface of the lid (16), that is, the lower surface plate (100). Thus, when the lid is placed in the on-position, the acting portion (112) acts on the actuator (110a) to place the humidifier in an operating state. In the fifth invention as well, by merely rotating the lid (16) to the on-position, it is possible to perform an operation.

A sixth invention is according to the fifth invention, and is an atomizing apparatus further comprising a first recessed portion and a second recessed portion which are provided on one of a top surface of the fixing plate and an undersurface of the lid at positions spaced apart, and a protruded portion which is provided on the other of the top surface of the fixing plate and the undersurface of the lid, and is engaged with the first recessed portion when the lid is in the on-position, and is engaged with the second recessed portion when the lid is in the off-position.

In the sixth invention, similar to the second invention, the lid (16) is rotated or turned between the on-position and the off-position with an operational feeling of click.

A seventh invention is according to any one of the first to sixth inventions, and is an atomizing apparatus, wherein the mist generator includes an ultrasonic wave vibrator.

In the seventh invention, the mist generator is constituted of an ultrasonic wave vibrator (76).

An eighth invention is according to the seventh invention, and is an atomizing apparatus, wherein the ultrasonic wave vibrator includes a vibrating portion, and further comprising: a first packing which has a sealing portion surrounding the vibrating portion on an undersurface of the ultrasonic wave vibrator, and a second packing which has a sealing portion surrounding the vibrating portion on an upper surface of the ultrasonic wave vibrator.

In the eighth invention, the vibrator (76) includes a vibrating portion (76a) at its central portion, and by a lower packing, that is, a sealing portion (74) of a first packing (70), the vibrating portion is surrounded at an undersurface of the vibrator. By an upper packing, that is, a sealing portion (88) of a second packing (78), the vibrating portion is surrounded at an upper surface of the vibrator. According to the seventh invention, when by vibrations of the vibrating portion (76a), mist is generated at this place, the vibrating portion is completely sealed by the sealing portion, so that the mist does not enter inside of the atomizing apparatus.

A ninth invention is according to the eighth invention, and is an atomizing apparatus wherein the second packing is formed with the opening, and further comprising a first sealing member which seals a surrounding of the opening of the second packing when the emitting opening is positionally superposed with the opening in the on-position.

In the ninth invention, when the opening (80) formed at the second packing is positionally superposed with the emitting opening (24) in the on-position, that is, while the atomizing apparatus is in operation, the surrounding of the opening of the second packing is sealed by the first sealing member, for example, the lower surface plate (100) of the lid (16). Accordingly, in the ninth invention, while the atomizing apparatus is in operation, mist never goes inside the tank from atmosphere around the opening.

A tenth invention is according to any one of the first to ninth inventions, and is an atomizing apparatus wherein the liquid storage has a closed-bottom cylindrical tank, and further comprising a sealer which prevents the liquid from leaking from the tank.

In the tenth invention, liquid, for example, water is stored in a closed-bottom cylindrical tank (40), and a sealer (41, 36a, 128) seals the water in the tank in a liquid-tight manner, and therefore, liquid for humidification never leaks even when the atomizing apparatus is carried.

An eleventh invention is according to the tenth invention, and is an atomizing apparatus wherein the sealer includes a third packing which is provided at an upper end of a side wall of the tank, and a second sealing member which seals an upper end of the third packing.

In the eleventh invention, the sealer includes a third packing (41) provided at an upper end of a side wall of the tank, and the upper end of the third packing is sealed by a sealing member (36a), such as a top plate of a cylindrical casing. According to the eleventh invention, when the tank is installed within the housing, the tank is sealed in a liquid-tight manner.

A twelfth invention is according to the tenth or the eleventh invention, and is an atomizing apparatus further comprising: a side wall portion which defines a space communicating with an inside of the tank, and a hole provided on the side wall portion, and a filter which is set on the hole, and can transmit air molecules but cannot transmit water molecules, wherein the filter is made up of a part of the sealer.

In the twelfth invention, a part of the sealer includes a filter (128) which is set on a hole (130) formed on a side wall portion (124b) which defines a space communicating with an inside of the tank.

A thirteenth invention is according to any one of the tenth to twelfth inventions, and is an atomizing apparatus, wherein the tank stores water including chloride ion, and further comprising at least a pair of electrolysis electrodes which are set within the tank and are used for performing electrolysis.

According to the thirteenth invention, by a pair of electrolysis electrodes (122p, 122m), electrolysis is performed within the tank. Thus, hypochlorous acid and hydroxyl radical are retained in a high concentration in the water including chloride ion (tap water) in the tank, and mist is generated from the electrolyzed water, and whereby, it is possible to expect a disinfection effect around the atomizing apparatus by the mist emitted for humidification.

A fourteenth invention is according to the thirteenth invention, and is an atomizing apparatus further comprising: a shortage of water detecting electrode which is set within the tank and has a lower end positioned higher than lower ends of the pair of the electrolysis electrodes, and a shortage of water detector which detects a shortage of water state within the tank by a current of the shortage of water detecting electrode when the electrolysis electrodes are energized.

In the fourteenth invention, the shortage of water detector (132), such as the computer can detect a shortage of water state, that is, a state in which a predetermined amount or more water is not within the tank depending on whether a current flows through the shortage of water detecting electrode (124) when the electrolysis electrodes are energized.

According to the present invention, it is possible to obtain a portable atomizing apparatus.

The above described objects and other objects, features, aspects and advantages of the present invention will become more apparent from the following detailed description of the present invention when taken in conjunction with the accompanying drawings.

### BRIEF DESCRIPTION OF THE DRAWINGS

Figure 1 is an illustrative view showing an appearance of one embodiment of the present invention, Figure 1 (A) is a font view, and Figure 1 (B) is a plan view.
Figure 2 is an illustrative front view with a housing removed in order to show an internal configuration of Figure 1 embodiment, and partially broken away so as to pass through the center of a releasing opening shown in Figure 1 (B).
Figure 3 is an illustrative view showing a vibrator portion of Figure 1 embodiment in detail.
Figure 4 is an illustrative view of a fixing plate provided at an upper end of the housing shown in Figure 1 embodiment as seen from above.
Figure 5 is an illustrative view showing a lower surface plate of a lid placed above the fixing plate in Figure 1 embodiment.
Figure 6 is an illustrative view showing a different positional relation between the fixing plate and the lower surface plate (lid) in Figure 1 embodiment.
Figure 7 is an illustrative view showing an electrode unit in Figure 1 embodiment.
Figure 8 is a side view showing the electrode unit shown in Figure 7.
Figure 9 is an illustrative view showing a part of an air passage of Figure 1 embodiment in detail.
Figure 10 is a block diagram showing a configuration of Figure 1 embodiment.

### BEST MODE FOR PRACTICING THE INVENTION

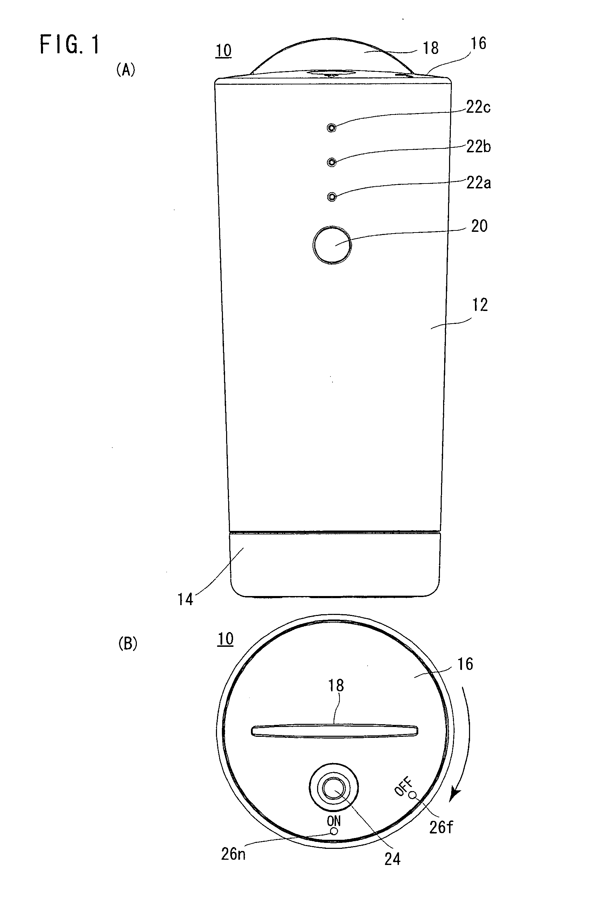

Referring to Figure 1, an atomizing apparatus 10 in this embodiment is basically operated by taking a rechargeable battery (not shown) as a power source, and is formed into a cylindrical shape being smaller in diameter toward the downward direction in the order of 10 cm in diameter and 20 cm in height as a whole. If the atomizing apparatus 10 is intended to be used in the car, the diameter is selected to fit in a hole-like cup holder formed on an inner surface of the doors of the car, or an annular cup holder attached below a dashboard (both not shown). Here, the atomizing apparatus 10 in this embodiment is portable, and can be freely set not only in the car but also on a desk or in a toilet.

Furthermore, it is to be understood that almost all of the components of the atomizing apparatus 10 of this embodiment to be explained below are plastic molded products unless otherwise annotated.

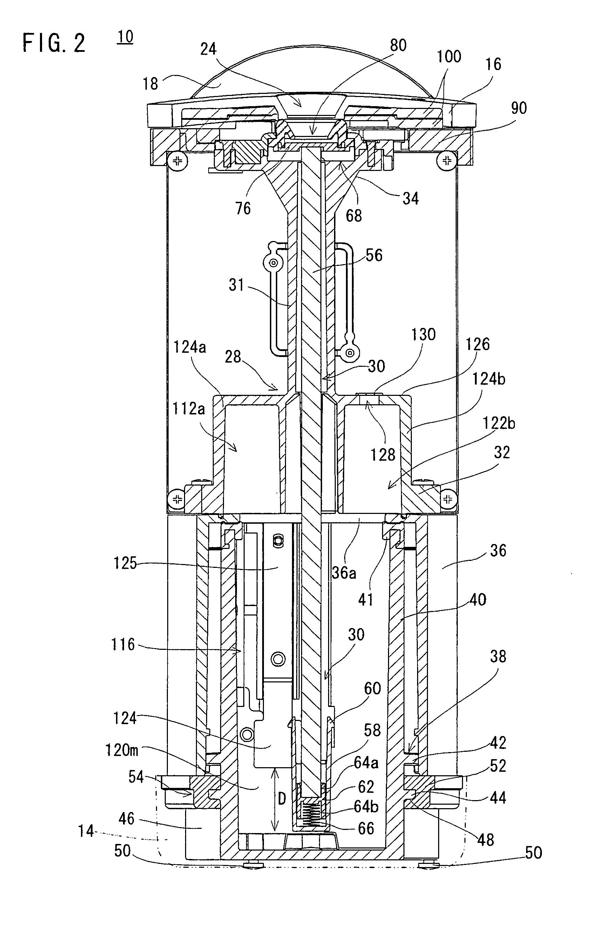

The atomizing apparatus 10 includes a housing 12 deciding the above-described diameter and having a height that extends the entire length of the apparatus. The housing 12 is opened at both upper and lower ends, and detachably attached with a waterproof cover 14 to cover a tank 40 (Figure 2) for water reservoir at a lower end. The waterproof cover 14 is integrated with the tank, and the waterproof cover 14 is rotated to thereby be attached to and detached from the housing 12 together with the tank. In the tank covered with the waterproof cover 14, liquid such as water (specifically tap water is suitable from the reason described later) is retained.

On an upper end of the housing 12, a lid 16 is rotatably attached. The lid 16 is provided with a knob 18 on its top surface, and the lid 16 can be rotated or turned with an operational feeling of click between an on-position and an off-position with the knob 18 held. That is, by rotating the lid 16, it is possible to control on-off actions of the atomizing apparatus 10.

On a front surface of the housing 12, a switch 20 is provided, and the switch 20 is a push button switch for changing an amount of atomizing, that is, the strength of humidification, and every time that the switch 20 is pushed, it is possible to alternately set a "strong" and a "weak". Here, in this embodiment, if the "strong" is set, mist is continuously diffused, but if the "weak" is set, mist is intermittently diffused, such as a one-second-ON and a 0.5-second-OFF. However, the setting between the strong and the weak is not restricted to that of this embodiment, and can take an arbitrary specification.

Above the switch 20 on the front surface of the housing 12, three LEDs 22a, 22b and 22c are arranged in a vertical direction to be aligned with the switch 20. The lowermost LED 22a is for displaying whether or not the aforementioned battery is charged, and lit when charged. The LEDs 22b and 22c are lit to individually display the "strong" and "weak" switched by the switch 20. Here, the LEDs 22b and 22c are two-color LEDs, for example, and also function as a shortage of water state displaying (notifying) means as described later.

The lid 16 is formed with an emission opening 24 for emitting mist as well understood from a plan view shown in Figure 1 (B). An "ON" display 26n is formed at the position aligned with the emission opening 24, and an "OFF" display is set at a position circumferentially spaced apart therefrom. When the lid 16 is rotated to a position (on-position) such that the "ON" display 26n is aligned with the switch 20, an atomizing operation is performed as described above.

Figure 2 is an illustrative view, partially broken away, of the inside of the atomizing apparatus 10 with the housing 12 shown in Figure 1 (A) removed. Inside the housing 12, a frame 28 having approximately the same height as the length of an upper half of the housing 12 is provided. The frame 28 has a side wall 31 for forming a hollow portion 30 below the emission opening 24 in Figure 1(B), and at a lower end of the frame 28, that is, at an approximately middle of the height of the atomizing apparatus 10, an attaching portion 32 is formed, and at an upper end of the frame, a placing portion 34 is formed.

To a bottom surface of the attaching portion 32, a cylindrical casing 36 which has a top plate 36a at an upper end and is opened at a lower end, although its front surface is cut out in Figure 2, is attached such that the top plate 36a is intimate contact with a bottom surface of the attaching portion 32 by a screw, for example. The casing 36 is for detachably loading a tank 40 as a water reservoir means, and is formed with a spiral groove 38 on its inner peripheral surface. On an outer peripheral surface of the tank 40, a spiral ridge 42 to threadedly be engaged with the groove 38 is formed.

On the outer periphery of the tank 40, an annular ridge 44 is formed below the aforementioned spiral ridge 42. The annular ridge 44 is engaged with an annular groove 48 formed on an inner peripheral surface of a packing 46 taking a shape of a cylinder with open ends and made of elastic material such as synthetic rubber. Although simply illustrated by a two-dotted line in Figure 2, the waterproof cover 14 shown in Figure 1 is secured to the outside of the packing 46 by a screw 50.

On an upper end of the packing 46, a flange 52 protruded outwardly as a sealing portion is formed, and the flange 52 is relatively thick in a radius direction, and on an inner surface of the flange 52, the above-described annular groove 48 is formed. On an outer surface of the sealing portion 52, a step 54 is formed. The step 54 is engaged with a step (not illustrated) formed on an upper inner surface of the waterproof cover 14, so that the waterproof cover 14 is securely attached to the packing 46.

Thus, the waterproof cover 14 is integrated with the packing 46, and the packing 46 and the tank 40 are integrated by the engaging portions, that is, the annular ridge 44 and the annular groove 48, so that the waterproof cover 14 and the tank 40 are eventually integrated. Accordingly, by rotating the waterproof cover 14, it is possible to rotate the tank 40 together therewith. Thus, by rotating the waterproof cover 14 counterclockwise (seen from a bottom surface of the atomizing apparatus 10), for example, the spiral ridge 42 can be released from the spiral groove 38 to thereby detach the tank 40 from the casing 36. On the contrary, by rotating the waterproof cover 14 clockwise (seen from the bottom surface of the atomizing apparatus 10), the spiral ridge 42 can be screwed into the spiral groove 38 to thereby attach the tank 40 to the casing 36. Accordingly, when water in the tank 40 is lost to be placed in a water-shortage state, the waterproof cover 14 may be rotated to detach the tank 40 from the casing 36, and after water (tap water) is replenished in the tank, the tank 40 is screwed into the casing 36 again with the waterproof cover 14 held. Thus, the tank 40 is in a form of a closed-bottom cylindrical cup, and the tank 40 with the tap water replenished is attached to the casing 36.

The tank 40 is covered with a ring-shaped packing 41 being made of elastic body like synthetic rubber at an upper end of a side wall. When the tank 40 is screwed into the casing 36, the packing 41 is strongly pressed against the top plate 36a of the casing 36. Thus, a sealing means for the tank 40 is made up of the packing 41 and the top plate 36a.

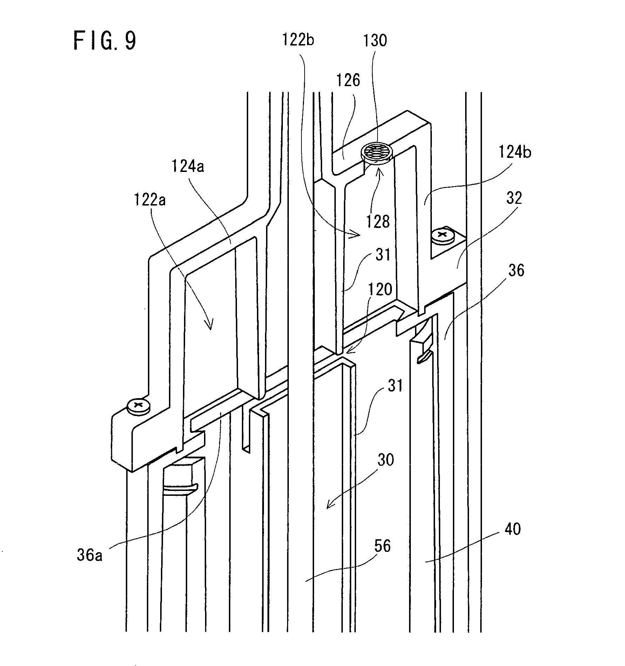

The hollow portion 30 of the frame 28, that is, the side wall 31 is also formed below the attaching portion 32 as especially understood from Figure 9, and thus, the hollow portion 30 is formed so as to extend from the upper end opening of the tank 40 toward the inside of the tank 40. A window (not illustrated) is formed at a part of the side wall 31 of the hollow portion below the attaching portion 32 (Figure 9). Accordingly, the hollow portion 30 provides a communication with the water (not illustrated) in the tank 40 through the window. That is, the water in the tank 40 permeates into the hollow portion 30.

The hollow portion 30 contains a feedwater rod 56 being made of felt (laminated and waulked fibers), for example, having a length approximately equal to the entire length of the height of the hollow portion. The felted material forming the feedwater rod 56 is a product made of laminated fibers, and thus exerts capillary function with respect to liquid. The water in the tank 40 permeates into the hollow portion 30 while the feedwater rod 56 is contained in the hollow portion 30, so that the water is supplied toward the upper potion of the feedwater rod 56 by the capillary function of the feedwater rod 56.

When holding structure of the feedwater rod 56 is described in detail, a closed-bottom cylindrical cap 58 is detachably attached to the lower end of the side wall of the hollow portion 30 such that an upper end of a wedge-shaped engaging portion 60 is engaged with an engaged portion (not illustrated) formed on the side wall of the hollow portion 30. Here, the side wall of the hollow portion to be formed with the engaged portion is not illustrated in Figure 2, but the side wall 31 is well illustrated in Figure 9.

Inside the cap 58, a holder 62 whose vertical cross-section is an "H" letter and having concave portions up and down is housed. An upper concave portion 64a of the holder 62 receives the lower end of the above-described feedwater rod 56, and the lower concave portion 64b contains a coil spring 66. The lower end of the coil spring 66 is in contact with the bottom surface of the cap 58, so that by the elastic repulsion force of the coil spring 66, the holder 62 is forced upwardly. Thus, the feedwater rod 56 whose lower end is supported by the holder 62 is forced upwardly.

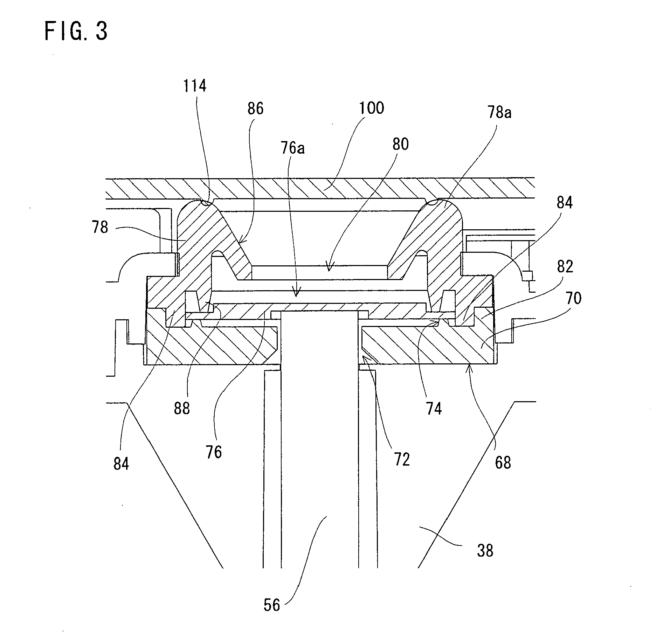

At the upper end of the frame 28, the placing portion 34 is formed as described above. A shape of the outer surface of the placing portion 34 is a funnel shape, and on the upper surface, a recessed portion 68 is formed. In the recessed portion 68, a donut-shaped lower packing 70 being made of elastic material such as synthetic rubber well seen from Figure 3 is placed. The aforementioned upper end of the feedwater rod 56 is presented to an opening 72 of the lower packing 70. Here, the position of the opening 72 corresponds to the position of the emission opening 24 shown in Figure 1 (B).

On the upper surface of the lower packing 70, a circumferential sealing portion 74 whose cross-section is acute triangular and whose plan view is donut shape is formed, and on the sealing portion 74, a disk-like ultrasonic wave vibrator (may be simply referred to as "vibrator") 76 as one example of a mist generator is placed. The vibrator 76 generates high frequency (ultrasonic wave) vibrations by applying a voltage to piezoelectric ceramics, and has a vibrating portion 76a at the center thereof. Since the detail of the vibrator of such a kind has been well known, a further explanation is omitted here.

The placing portion 68 is formed on the hollow portion 30, and at the placing portion 68, the vibrator 76 is placed on the lower packing 70, so that the vibrator 76 is positioned on the hollow portion 30, that is, the feedwater rod 56.

An upper packing 78 having an opening 80 formed at a position overlaid with the position of the opening 72 of the lower packing 70 and being made of elastic material such as synthetic rubber is integrally assembled with the lower packing 70 through adhesion by an adhesive. That is, at an upper surface of an outer periphery of the lower packing 70, an annular protrusion 82 formed to be projected upward is formed, and an outer peripheral surface of an annular protrusion 84 formed to be projected downward from a bottom surface of the outer periphery of the upper packing 78 is closely engaged with an inside peripheral surface of the protrusion 82, and a ridge formed on an upper surface of the protrusion 82 and a groove formed on a bottom surface of the protrusion 84 are engaged with each other, so that the lower packing 70 and the upper packing 78 are integrated tightly.

The opening 80 of the upper packing 78 is defined by a funnel-shape tilted portion 86 which is tilted inward as it going down, and the center of the tilted portion 86 is formed as the opening 80.

Between the aforementioned tilted portion 86 of the upper packing 78 and the aforementioned protrusion 84, a sealing portion 88 whose cross-section is triangular and whose plan view is annular is formed to be projected downward. As described above, the vibrator 76 is placed on the sealing portion 74 of the lower packing 70, and thus, the top of the sealing portion 74 is brought into engagement with a bottom surface of the vibrator 76. On the other hand, as described above, the top of the sealing portion 88 of the upper packing 78 projected downward is brought into contact with a top surface of the vibrator 76 at a position more inward than the position where the sealing portion 74 is brought into contact with the bottom surface. That is, the vibrator 76 is securely retained on the placing portion 68 by being sandwiched by the sealing portions 88 and 74.

As described above, since the feedwater rod 56 is forcedly upward by the coil spring 66 (Figure 2), the upper end of the feedwater rod 56 is securely brought into contact with the central bottom surface of the vibrator 76 through the opening 72 of the lower packing 70. The feedwater rod 56 gradually and continuously sucks up water in the tank 40 because it is made of a felted material, for example as described above. The water that reaches the upper end of the feedwater rod 56 is converted into mist by the vibrator 76, and the mist is emitted (atomized) through the opening 80 of the upper packing 78 and the emitting opening 24 of the lid 16 to exert a humidifying function. Here, the emitting opening 24 is subject to be positionaly overlapped with the opening 80.

As shown in Figure 3 and explained above, the vibrating portion 76a of the vibrator 76 is surrounded by the upturned sealing portion 74 of the lower packing 70 on the bottom surface of the vibrator 76, and the vibrating portion 76a is surrounded by the downturned sealing portion 88 of the upper packing 78 on the upper surface, so that it is possible to surely prevent leakage of the water on the top and the bottom surfaces of the vibrator 76 and undesired emission of mist from occurring.

For example, assuming that the force pushing up the feedwater rod 56 by the coil spring 66 is F1, and the force applied from the sealing portion 88 of the upper packing 78 to retain the vibrator 76 against the lower packing 70 is F2, each of forces is set so as to satisfy a relation of F2>F1, whereby it is possible to surely retain the vibrator 76 against the lower packing 70 and prevent water leakage from occurring.

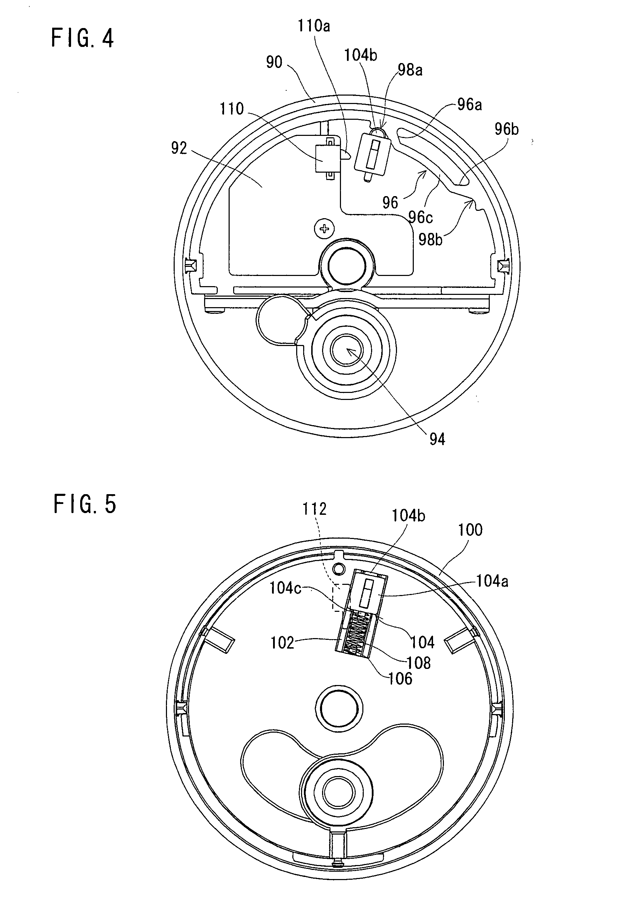

At the upper end of the frame 28, a fixing plate 90 shown in a plan view in Figure 4 is secured. On the fixing plate 90, a circuit board 92 is attached and on the circuit board 92, electronic components for making up of the electric circuit is mounted and wired in a predetermined manner, but these are not illustrated in Figure 4 in order to make the drawings simple.

On the fixing plate 90, an opening 94 is formed at a position overlaid on the aforementioned opening 80 (Figure 3). Accordingly, mist is finally emitted or atomized to the outside through the opening 80, the opening 94 and the emitting opening 24.

On the upper surface of the fixing plate 90, a rotation guide 96 for implementing a step-by-step rotation (click stop) the lid 16 as described is formed. The guide 96 is a communication member, formed in a predetermined circumferential position closer to an outer periphery of the fixing plate 90, includes slanting sides 96a and 96b on both sides and a connecting side 96c sandwiched between the slanted sides 96a, 96b. The connecting side 96c is curved such that the both ends are at slightly inward positions and the center is at an outward position like the outer peripheral edge of the fixing plate 90. In addition, outside root ends (outer periphery sides) of the slanting sides 96a and 96b, recessed portions 98a and 98b are formed. Conversely, the recessed portions 98a and 98b are communicated by the guide 96.

As well understood from Figure 2, on the bottom surface of the lid 16, a two-layer lower surface plate 100 is assembled so as to be integrated with the lid 16. The lower surface plate 100 may be originally integrally produced with the lid 16, but this is separately prepared from the lid 16 for facilitating the injection molding. In this sense, the lower surface plate 100 can be regarded as a part of the lid 16.

As shown in Figure 5, on the lower surface plate 100, that is, the bottom surface of the lid 16, a concave portion 102 whose shape is approximately a rectangle and whose longitudinal direction is along a radial direction from the center is formed. On the concave portion 102, a following member 104 is installed. The following member 104 includes a main body 104a, a protruded portion 104b whose tip end is circular and which is provided at a leading end of the main body 104a, and a lug 104c which is provided at a trailing end of the main body 104a, and between the lug 104c and a lug 106 formed on a bottom side of the concave portion 102, a coil spring 108 is interposed. Accordingly, the main body 104a of the following member 104, that is, the protruded portion 104b is always elastically energized by the coil spring 108 forward. Thus, the protruded portion 104b is pressed against the above-described rotation guide 96 provided in front thereof.

The fixing plate 90 shown in Figure 4 and the lower surface plate 100 shown in Figure 5 are placed in a corresponding positional relation; and this is equivalent to a situation in which the "ON" display 26n on the lid 16 shown in Figure 1 (B) is placed in the on-position, that is, placed to be aligned with the switch 20 shown in Figure 1(A). At this time, the leading end protruded portion 104b of the following member 104 is engaged in the one recessed portion 98a formed outside the both ends of the guide 96. The main body 104a of the following member 104 is in a state pressed forward by the coil spring 108, so that the lid 16 is retained at this position.

On the other hand, on the circuit board 92 (Figure 4), a limit switch 110 functioning as a operating switch is attached, and an actuator 110a of the limit switch 110 is projected from the circuit board 92 toward the guide 96.

An acting portion 112 represented by dotted line in Figure 5 is a protrusion projected downward from the bottom surface of the lower surface plate 100, and acts on the actuator 110a of the limit switch 110 at the on-position shown in Figure 4 and Figure 5 to push it. Accordingly, at this on-position, the limit switch 110 is placed in an on-state, so that the atomizing apparatus 10 is placed in an activating state, that is, an operating state as described later.

When from the on-state, the lid 16 is rotated with the knob 18 held by a certain degree or more force in an arrow direction shown in Figure 1 (B), the protruded portion 104b of the following member 104 is disengaged from the recessed portion 98a and moves along the slanting side 96a, abutting the inner surface of the slanting side 96a. Since in this situation, the pressing of the actuator 110a of the limit switch 110 by the protrusion of the lid 16, that is, the acting portion 112 is released, and the limit switch 110 becomes the off-state, so that the operation of the atomizing apparatus 10 is stopped.

Since the slanting side 96a of the guide 96 is inclined inwardly, the protruded portion 104b of the following member 104, that is, the main body 104a is pressed backward against the elastic force of the coil spring 108, and in accordance with a continual rotation of the lid 16, the protruded portion 104b of the following member 104 moves with reposing on the connecting side 96c of the guide 96. At this time, the coil spring 108 is in the most compressed state.

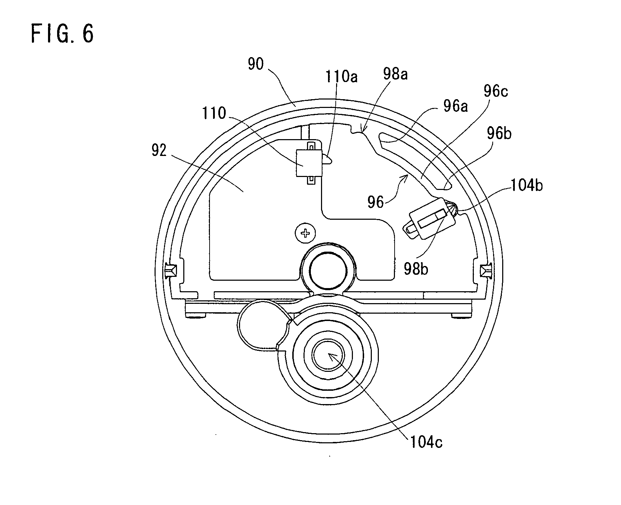

When the lid 16 is further rotated or continuously rotated, the protruded portion 104b of the following member 104 moves along the other slanting side 96b of the guide 96. Accordingly, the compression of the coil spring 108 is gradually released to press forward the main body 104 of the following member 104. In accordance with the continuation of the rotation, the leading end protruded portion 104b of the following member 104 is engaged with the other recessed portion 98b, snaps down there by the pressure of the coil spring 108, and then, the rotation of the lid 16 is stopped, and the lid 16 is retained at this position. The situation is an off-state in which the "off" display 26f of the lid 16 is aligned with the switch 20. The positional relation between the protruded portion 104b of the following member 104 in the off-state and the guide 96 is shown in Figure 6.

Thus, the lid 16 is rotated in a step-by-step manner and switched between ON and OFF by the spring 108, the following member 104, that is, the protruded portion 104b, and the guide 96 including the recessed portions 98a, 98b functioning as a follower. Then, on both states, the on-state and the off-state, the protrusion is engaged with each of the recessed portions, so that it is possible to securely retain the on-state and the off-state. Accordingly, even if the atomizing apparatus 10 of this embodiment is held in a cup holder (not illustrated) in a car, an undesirable changeover between the on-state and the off-state due to vibrations transmitted from the car can be prevented.

Here, as to the recessed portions 98a, 98b and the protruded portion 104b, on the contrary to the embodiment shown in Figure 4 and Figure 5, the recessed portions 98a, 98b are formed on the lid 16, that is, the lower surface plate 100, and the protruded portion 104b to be engaged therewith may be formed on the fixing plate 90.

Returning to Figure 3, the force F2 that the aforementioned upper packing 78 applies to the vibrator 76 is maintained by the lower surface plate 100 of the lid 16. That is, at a position corresponding to the top portion 78a of the upper packing 78 on the bottom surface of the lower surface plate 100, a downward annular ridge 114 is formed. Then, when the annular ridge 114 is pressed against the top portion 78a of the upper packing 78 by the lid 16, the aforementioned F2 is applied to the upper packing 78, and a water seal between the upper packing 78 and the lower surface plate 100, that is, the lid 16 is implemented, capable of efficiently preventing water leakage therefrom from occurring. Here, as shown in Figure 3, the seal of the top portion 78a of the upper packing 78 by the ridge of the lower surface plate 100 may be implemented only when the lid 16 is rotated to the on-position. When the lid 16 is placed in the off-position, the operation is in a shut-down state, and the mist is not generated, so that the mist never enters the housing 12 (Figure 1) even if the opening 80 is not sealed. In addition, the pressure of the lid 16 against the lower surface plate 100 is given by a holding means (not illustrated) for securely holding the lid 16 and an integrating means (not illustrated) such as a screw for integrating the lid 16 and the lower surface plate 100.

The atomizing apparatus 10 shown in Figure 1 embodiment further includes other features, one of which is that by performing electrolysis processing on water including chloride ion like tap water accumulated in the tank 40 to produce electrolyzed water, and by dissipating the electrolyzed water as mist, this is directed to exert a function as a humidifier and an effect of suppression of viruses (disinfection) as well. That is, by performing electrolysis on the tap water in the tank 40 to generate two kinds of active oxygen for suppressing virus, such as "hypochlorous acid" and "hydroxyl radical", retaining the active oxygen in a high concentration in the water in the tank 40, and emitting it with water as mist, this is directed to exert an effect of disinfection around the atomizing apparatus 10 (http://www.sanyo.co.jp/vw/concept/index.html).

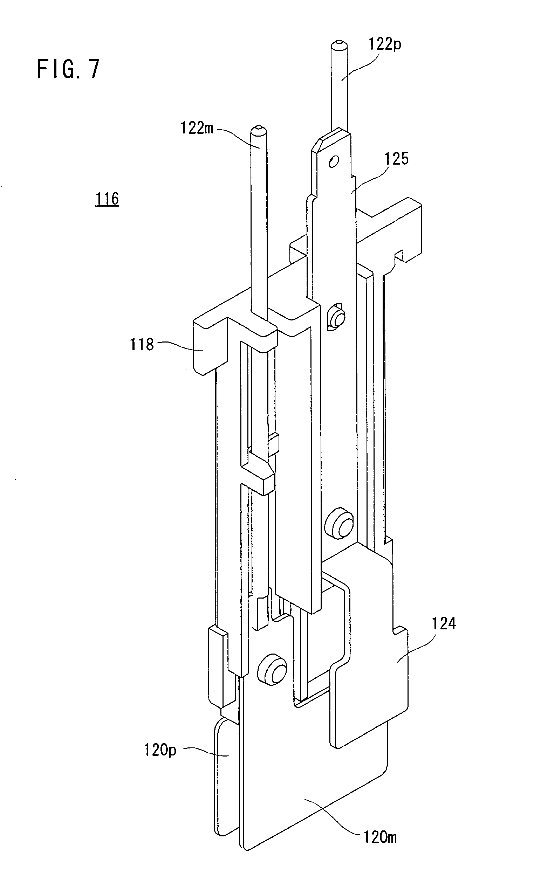

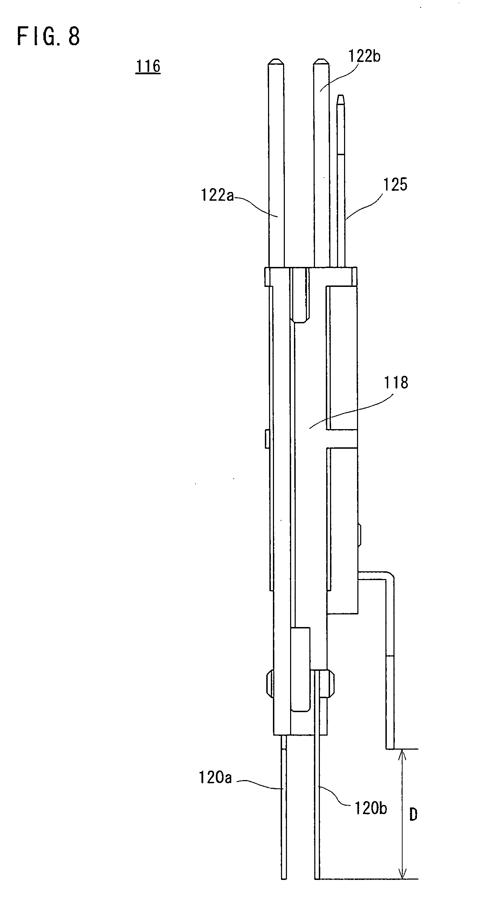

For such electrolysis processing, an electrode unit 116 shown in Figure 7 and Figure 8 is utilized in this embodiment.

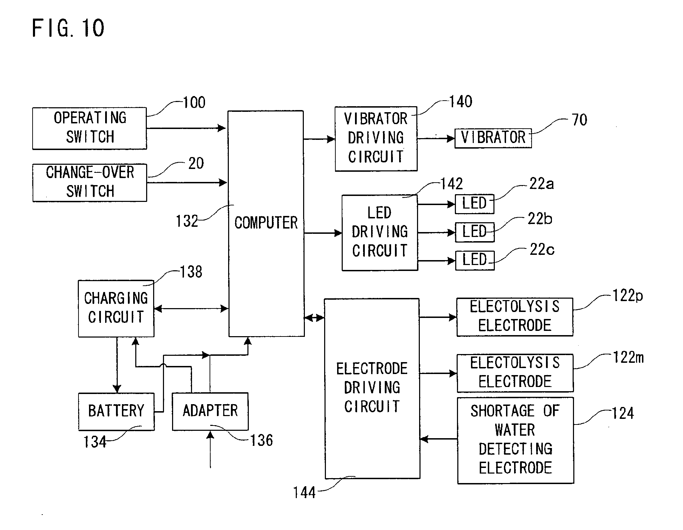

The electrode unit 116 takes approximately the shape of rectangle, and includes a holding plate 118 being formed with an electrode holding portion necessary for holding the electrodes such as grooves and protrusions on both sides. On the one main surface of the holding plate 118, an electrode 120p as a positive electrode is attached by an appropriate means, such as by a screw or by swaging, for example. On the other main surface of the holding plate 118, an electrode 120m as a negative electrode is attached by a similar attaching means. The electrodes 120p and 120m are made of electrically conducting material, such as copper, to be worked into a planar shape, are connected to an electrode driving circuit 144 (Figure 10) described later by round-bar shaped connecting members 122p and 122m, respectively, each being made up of similar electrically conducting material, and receive direct-current power supply from the driving circuit to cause electrolysis.

The electrode unit 116 is attached with a shortage of water detecting electrode 124 that is made of electrically conducting material similar to the above-described electrodes 120p and 120m but separately set, on the other main surface of the holding plate 118 in parallel with the electrode 120m in this embodiment. The third electrode 124 is for detecting whether or not a certain amount of water or more is accumulated in the tank 40, and has a lower end positioned higher than the lower ends of the electrodes 120p and 120m by a distance D as specifically well understood from Figure 8. The shortage of water detecting electrode 124 is connected to the aforementioned electrode driving circuit 144 by a planar connecting member 125.

Such an electrode unit 116 is installed below the bottom surface of the attaching portion 32 of the frame 28 as shown in Figure 2, and in this state as well, the shortage of water detecting electrode 124 is attached in such a position that its lower end is higher than the lower ends of the electrolysis electrodes 120p and 120m by the distance D. In electrolysis, a DC voltage is applied across the two electrodes 120p and 120m as well known, and if the electrode 124 is under water at that time, a closed circuit is formed as well between the electrode 120p and the shortage of water detecting electrode 124 when the voltage is applied. However, if the shortage of water detecting electrode 124 is not under water, even if a voltage is applied to the electrode 120p, an electric circuit is not formed between the electrodes 120p and 124. Thus, by detecting whether or not a current flows into the shortage of water detecting electrode 124 when a voltage is applied to the electrode 120p, it is possible to detect whether or not a certain amount of water or more remains in the tank 40. A shortage of water detector which detects a shortage of water state within the tank 40 by the current flowing into the shortage of water detecting electrode 124 is a computer 132 (Figure 10).

As clearly understood, the "predetermined amount" is set by the aforementioned distance D, and by setting the distance D at will, it is possible to arbitrarily set the predetermined amount. The state that the water in the tank 40 is below the predetermined amount is called a shortage of water state, and therefore, the electrode 124 is a shortage of water detecting electrode.

In the atomizing apparatus 10 of this embodiment, as described before, the tank 40 is screwed into the casing 36 with the tank 40 with water. At this time, the tank 40 is not entirely filled with water, and has naturally air above the water. If the tank 40 is screwed into the casing 36 in that state, the remaining air is compressed to sometimes make the water overflow from the tank 40.

Hereupon, in the atomizing apparatus of this embodiment, an air passage as shown in Figure 9 is formed to prevent the water from overflowing when the tank 40 is attached.

Figure 9 is illustration for showing the surrounding of the attaching portion 32 of the frame 28 shown in Figure 1, and the casing 36 is mounted to the attaching portion 32, and the tank 40 is installed in the casing 36. The side wall 31 of the hollow portion 30 for housing the feedwater rod 56 is actually divided up and down by a gap 120 formed due to the top plate 36a of the casing 36 attached to the attaching portion 32 as shown in Figure 9 in detail. The gap 120 is formed on the walls 31 on both sides of the hollow portion 30 which sandwich the feedwater rod 56. In addition, side walls 124a and 124b for defining the spaces 122a and 122b are formed on the attaching portion 32, and on a ceiling 126 of the one side wall 124b, an air hole 128 is formed. The air hole 128 is provided with a filter 130. Although it is roughly drawn for the sake of illustration, a mesh size of the filter 130 is set to such a size that air molecules can be transmitted but water molecules cannot be transmitted.

When the tank 40 is screwed into the casing 36, the air within the tank 40 is gradually compressed, passes through the opening at the upper end of the tank 40, and finally goes through the air hole 128 to outside. At this time, in Figure 9, the air going out from the left side of the opening of the tank 40 flows into the space 122b through the gap 120 of the side wall 31 of the hollow portion 30, and goes out through the air hole 128. In Figure 9, the air going out from the right side of the opening of the tank 40 directly flows into the space 122b and goes out through the air hole 128. That is, in this embodiment, the air in the tank 40 is always communicated with the atmosphere through the passage including the gap 120, the space 122 and the air hole 128, so that a phenomena that a remaining air is compressed when the tank 40 is attached does not occur, capable of preventing the inconvenience of overflowing of the water in the tank 40 occurring.

Since the aforementioned filter 130 is set to the air hole 128, even if the water in the tank 40 overflows in the spaces 122a, 122b due to big shakes of the car, the water never spills through the air hole 128.

An electronic circuit configuration of the atomizing apparatus 10 of such structure is shown in a block diagram shown in Figure 10. Referring to Figure 10, the atomizing apparatus 10 includes the computer 132 for controlling an entire electronic operation of the atomizing apparatus 10, and the computer 132 is supplied with direct-current power from a battery 134 or an adapter 136. Although roughly illustrated in Figure 10, a direct-current power supply from an adapter 136 is also applied to a charging circuit 138. Thus, the battery 134 is charged by the charging circuit 138. Although not illustrated in Figure 2, the battery 134 is loaded at a position hidden in Figure 2 above the attaching portion 32 of the frame 28.

Here, a less current is flown through the computer 132 even in an off-state of the operating switch 110 to make the computer 132 persist a standby state, and also causes the data in the internal memory (not shown) of the computer 132 to be held in the memory.

The computer 132 is connected with the limit switch 110 shown in Figure 4 as an operating switch, and also connected with the changing-over switch 20 (Figure 1).

The computer 132 instructs a vibrator driving circuit 140 to perform on-off control of the vibrator 76. The computer 132 further instructs an LED driving circuit 142 to make on-off control of the LEDs 22a, 22b and 22c.

The computer 132 controls an electrode driving circuit 144. The electrode driving circuit 144 applies a direct voltage for electrolysis across the electrolysis electrodes 120p and 120m. The computer 132 further detects whether or not a current flows into the shortage of water detecting electrode 124 on the basis of a signal from the electrode driving circuit 144.

When a rotation of the lid 16 to the on-position makes the acting portion 112 act on the actuator 110a of the limit switch 110, to thereby turn the limit switch, that is, the operating switch 110 on, the computer 132 instructs the vibrator driving circuit 140 to control the vibrator 76 to an on-state. Thus, an atomizing operation by the atomizing apparatus 10 is performed.

The computer 132 instructs the electrode driving circuit 142 to apply a direct voltage for electrolysis between the electrolysis electrodes 122p and 122m as necessary.

Here, although not explained above, the charge of the battery 134 (Figure 10) is constantly executed when the remaining amount of the battery is lowered and the direct voltage is supplied by the adapter 136 (Figure 10), even if the operating switch 110 is turned off, or even if the shortage of water state is detected.

In the above-described embodiment, by using one shortage of water detecting electrode 124, a shortage of water state is detected. However, by using a plurality of water detecting electrodes (shortage of water detecting electrodes) other than the electrolysis electrodes, and by setting the level (more specifically, one being equivalent to a distance D between the lower end of each water detecting electrode and the lower end of the electrolysis electrode (Figure 8)) of each electrodes stepwise, the amount of the water in the tank 40 may be determined stepwise. In this case, depending on the detected amount of water, an initial electrolysis operating time is changed to thereby generate electrolyzed water of more optimal concentration. Although detailed explanation is omitted, in the above-described embodiment, in order to maintain the concentration of the electrolyzed water at a necessary concentration, electrolysis processing is executed as a initial electrolysis operation during a predetermined time period at a start of the operation. Here, if such an initial electrolysis operating time is set in correspondence with the amount of water in the tank 40, it is possible to more surely maintain the electrolyzed water concentration at an optimal level.

Although the present invention has been described and illustrated in detail, it is clearly understood that the same is by way of illustration and example only and is not to be taken by way of limitation, the spirit and scope of the present invention being limited only by the terms of the appended claims.

## Claims

1. An atomizing apparatus comprising:
a housing;
a liquid storage which stores liquid for humidification within said;
a liquid supplier which supplies liquid stored said liquid storage upward;
a mist generator which is provided at an upper end of said housing, and is supplied with said liquid from said liquid supplier to generate mist from said liquid;
an opening which is formed above said mist generator, and passes the mist generated by said mist generator;
a lid which is provided to be rotated between an on-position and an off-position on an upper end of said housing, and
an emitting opening which is provided on said lid, and communicated with said opening when said lid is rotated to said on-position, wherein
said mist is emitted through said opening and said emitting opening.

2. An atomizing apparatus according to claim 1, further comprising:
a fixing plate which is provided below said lid at an upper part of said housing;
a first recessed portion and a second recessed portion which are provided on one of a top surface of said fixing plate and an undersurface of said lid at positions spaced apart, and
a protruded portion which is provided on the other of the top surface of said fixing plate and the undersurface of said lid, and is engaged with said first recessed portion when said lid is in said on-position, and is engaged with said second recessed portion when said lid is in said off-position.

3. An atomizing apparatus according to claim 2, further comprising:
a communicating member which provides communication between said first recessed portion and said second recessed portion, wherein
said communicating member includes a slanting side being inclined inwardly near said first recessed portion and said second recessed portion and a connecting side connecting said slanting sides,
a follower which follows along said communicating member by displacing said protruded portion.

4. An atomizing apparatus according to claim 1, further comprising a switcher operated when said lid is placed in said on-position.

5. An atomizing apparatus according to claim 4, further comprising a fixing plate which is provided below said lid at an upper part of said housing, wherein
said switcher includes a switch which is provided on one of a top surface of said fixing plate and an undersurface of said lid and has an actuator, and an acting portion which is provided on the other of the top surface of said fixing plate and the undersurface of said lid and acts on said actuator when said lid is placed in said on-position.

6. An atomizing apparatus according to claim 5, further comprising:
a first recessed portion and a second recessed portion which are provided on one of a top surface of said fixing plate and an undersurface of said lid at positions spaced apart, and
a protruded portion which is provided on the other of the top surface of said fixing plate and the undersurface of said lid, and is engaged with said first recessed portion when said lid is in said on-position, and is engaged with said second recessed portion when said lid is in said off-position.

7. An atomizing apparatus according to any one of claims 1 to 6, where said mist generator includes an ultrasonic wave vibrator.

8. An atomizing apparatus according to claim 7, wherein
said ultrasonic wave vibrator includes a vibrating portion, and further comprising:
a first packing which has a sealing portion surrounding said vibrating portion on an undersurface of said ultrasonic wave vibrator, and
a second packing which has a sealing portion surrounding said vibrating portion on an upper surface of said ultrasonic wave vibrator.

9. An atomizing apparatus according to claim 8, wherein
said second packing is formed with said opening, and further comprising a first sealing member which seals a surrounding of said opening of said second packing when said emitting opening is positionally superposed with said opening in said on-position.

10. An atomizing apparatus according to any one of claims 1 to 9, wherein said liquid storage has a closed-bottom cylindrical tank, and further comprising a sealer which prevents said liquid from leaking from said tank.

11. An atomizing apparatus according to claim 10, wherein said sealer includes a third packing which is provided at an upper end of a side wall of said tank, and a second sealing member which seals an upper end of said third packing.

12. An atomizing apparatus according to claim 10 or 11, further comprising:
a side wall portion which defines a space communicating with an inside of said tank, and
a hole provided on said side wall portion, and
a filter which is set on said hole, and can transmit air molecules but cannot transmit water molecules, wherein
said filter is made up of a part of said sealer.

13. An atomizing apparatus according to any one of claims 10 to 12, wherein said tank stores water including chloride ion, and further comprising
at least a pair of electrolysis electrodes which are set within said tank and are used for performing electrolysis.

14. An atomizing apparatus according to claim 13, further comprising:
a shortage of water detecting electrode which is set within said tank and has a lower end positioned higher than lower ends of said pair of the electrolysis electrodes, and
a shortage of water detector which detects a shortage of water state within said tank by a current of said shortage of water detecting electrode when said electrolysis electrodes are energized.
